**Europäisches Patentamt**

(19) **European Patent Office**

**Office européen des brevets**

(11) Veröffentlichungsnummer: **0 151 758**

**A2**

(12) # EUROPÄISCHE PATENTANMELDUNG

(21) Anmeldenummer: 84115508.8

(51) Int. Cl.⁴: **A 61 K 6/04**
**A 61 C 5/04**

(22) Anmeldetag: 15.12.84

(30) Priorität: 03.02.84 DE 3403779

(43) Veröffentlichungstag der Anmeldung:
21.08.85 Patentblatt 85/34

(84) Benannte Vertragsstaaten:
AT BE CH DE FR GB IT LI NL SE

(71) Anmelder: Degussa Aktiengesellschaft
Weissfrauenstrasse 9
D-6000 Frankfurt am Main 1(DE)

(72) Erfinder: Beyer, Hans-Hermann, Dr. Dipl.-Chem.
Am Birkenanger 11
D-8756 Kahl(DE)

(72) Erfinder: Diehl, Walter, Dr. Dipl.-Phys.
Gustav-Adolf-Strasse 29
D-6450 Hanau 1(DE)

(72) Erfinder: Eckert, Karlheinz, Dipl.-Ing.
Wibaustrasse 2
D-6466 Gründau 2(DE)

(72) Erfinder: Eiermann, Kurt, Dr. Dipl.-Phys.
Friedhofstrasse 26
D-6102 Pfungstadt 2(DE)

(72) Erfinder: Ringelstein, Hans-Martin
Inheidenerstrasse 22
D-6000 Frankfurt 60(DE)

(54) **Verfahren und Vorrichtung zur Herstellung von Füllungen in Zähnen.**

(57) Zur Herstellung von Füllungen in Zähnen mittels Stopfgold wird Goldpulver in die Kavität eingebracht und dieses anschließend mechanisch verfestigt. Die Feuchtigkeitsempfindlichkeit dieses Verfahrens kann man vermeiden, wenn man eine Paste aus plättchenförmigen Goldpulver mit einem bei 20 bis 45° C sich verflüssigenden, plastischen organischen Bindemittel verwendet und die mechanische Verfestigung unter Einwirkung von Ultraschall erfolgt.

0151758

84 106 DT

Degussa Aktiengesellschaft
Weissfrauenstraße 9, 6000 Frankfurt/Main

<u>Verfahren und Vorrichtung zur Herstellung von Füllungen in Zähnen</u>

Die Erfinder betrifft ein Verfahren zur Herstellung von Füllungen in Zähnen durch Einbringung von Goldpulver in die Kavität und anschließender mechanischer Verfestigung und eine Vorrichtung zur Durchführung dieses Verfahrens.

In der konservierenden Zahnheilkunde sind eine Reihe von metallischen Füllstoffen bekannt, wie beispielsweise Amalgame, Gußlegierungen in Form in Inlays oder Stopfgold. Die Füllung der Zahnkavitäten mit Stopfgold ist eine der ältesten Zahnfüllungsmethoden. Für diese Goldstopffüllungen wird chemisch reines Gold in Form von Goldfolie, Goldschwamm oder Goldpulver verwendet.

Die aus reinem Gold hergestellten Goldstopffüllungen werden in Bezug auf Haltbarkeit, Aestethik und Korrosionsbeständigkeit ausgezeichnet beurteilt. Schwerwiegende Nachteile der Goldstopffüllungen sind jedoch das technisch und zeitlich sehr aufwendige Präparieren der Kavität und das ebenfalls großes Geschick erfordernde Legen der Füllung. So ist zunächst eine sehr sorgfältige Bearbeitung der Kavität mit Unterschnitten und eine nicht automatisch durchführbare Aufrauhung der Kavitätenwände nötig. Beides ist unbedingte Voraussetzung für eine ausreichende Haftung des Goldes in der Kavität. Desweiteren muß die Kavität während des Goldstopfvorganges absolut frei von Feuchtigkeit sein. Dies betrifft nicht nur den Speichelfluß, sondern auch die Atemluft des Patienten. Dies macht die ebenfalls zeitraubende und für den Patienten mitunter sehr unangenehme Anwendung von sogenannten Cofferdam-Folien erforderlich.

Darüberhinaus muß das Material der Goldstopffüllung unmittelbar vor dem Einbringen in die Kavität in einer sehr sauberen Alkoholflamme ausgeglüht werden, damit sämtliche Verunreinigungen auf der Oberfläche entfernt werden und eine kohäsive Bindung zwischen den einzelnen Goldteilchen erreicht wird. Die Kaltschweissbarkeit des Goldes, die Grundlage des Goldstopfens, wird durch eine Kontamination der Oberfläche, insbesondere durch Flüssigkeitsfilme, sehr stark vermindert.

Aus der DE-OS 30 42 008 ist ein Goldstopfverfahren bekannt, bei dem ein poröser Sinterkörper oder ein Drahtgeflechtknäuel, vorzugsweise aus Gold, zusammen mit einem plastischen oder flüssigen organischen Bindemittel in die Kavität eingebracht und mit manuellen Stopfgeräten der Kavität angepasst und verfestigt wird. Mit diesem Verfahren lassen sich allerdings keine einwandfreien Oberflächen erzeugen, die außerdem nicht rein metallisch sind, sondern noch den organischen Binder, meist Methacrylate, enthalten.

Es war daher Aufgabe der vorliegenden Erfindung, ein Verfahren zur Herstellung von Füllungen in Zähnen zu entwickeln, durch Einbringung von Goldpulver in die Kavität und anschließender mechanischer Verfestigung, das nicht empfindlich auf Feuchtigkeit ist, ein rasches Arbeiten erlaubt und einwandfreie metallische Oberflächen liefert.

- 3 -

0151758

Diese Aufgabe wurde erfindungsgemäß dadurch gelöst, daß ein plättchenförmiges Goldpulver verwendet wird, das mit einem bei 20 bis 45° C sich verflüssigenden, plastischen organischen Bindemittel zu einer Paste verarbeitet wird, und daß die mechanische Verfestigung unter Einwirkung von Ultraschall erfolgt.

Vorzugsweise verwendet man als Bindemittel Polyäthylenglycol mit einem Molekulargewicht von 600 bis 1500 in Mengen von 0,5 bis 5 Gew%. Als sehr vorteilhaft hat sich ein Zusatz von rund 2,6 Gew% Polyäthylenglycol erwiesen. Die Goldplättchen haben vorteilhafterweise eine Größe von 5 bis 100 μm, bei einer Dicke von 0,1 bis 5 μm.

Aus dem plättchenförmigen Goldpulver und dem organischen Bindemittel, das physiologisch unbedenklich sein muß, wird eine Paste hergestellt und diese mit einem Werkzeug in die Kavität gebracht, wo sie unter Druck- und Ultraschalleinwirkung der Kavität angepasst und verfestigt wird. Überschüssiges Bindemittel wird dabei abgesondert und kann entfernt werden. Vorteilhafterweise werden wasser- bzw. speichellösliche Bindemittel verwendet.

Vorzugsweise verwendet man für das erfindungsgemäße Verfahren eine Vorrichtung, die aus einem Ultraschallerzeuger mit stabförmiger Sonotrode besteht. Die Sonotrode ist dabei von einer vor- und rückschiebbaren Hülse umgeben. Schiebt man die Hülse über die Spitze der Sonotrode hinaus, kann man mit dem überstehenden Teil der Hülse die Paste aufnehmen und nach dem Zurückschieben diese mit der Sonotrode in der Kavität

- 4 -

0151758

verdichten. Überraschenderweise eliminiert die Ultraschalleinwirkung vollständig den Einfluß von Feuchtigkeit auf die Verschweißbarkeit der Goldpaste, sodaß die Füllungen sogar unter Wasser oder Speichel gelegt werden können.

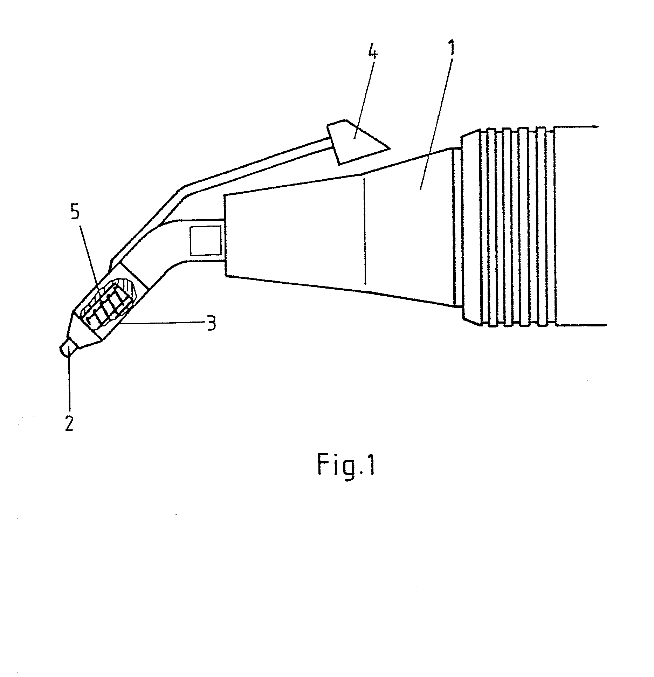

Die Abbildung zeigt schematisch einen Querschnitt durch eine solche Vorrichtung in beispielhafter Ausführungsform. Auf dem Ultraschallerzeuger (1) ist eine stabförmige Sonotrode (2) befestigt, die mit einer verschiebbaren Hülse (3) umgeben ist. An der Hülse (3) ist ein Hebel (4) befestigt, der es mit Hilfe der Rückholfeder (5) erlaubt, die Hülse (3) vor- und zurückzuschieben.

Folgendes Beispiel soll die Erfindung näher erläutern:

Plättchenförmiges Goldpulver ($15 \times 0{,}3\ \mu\text{m}^2$) wird mit 2,6 Gew% Polyäthylenglycol (Molekulargewicht ca 800, Fp: ca. $28^{\circ}$ C) angepastet. Diese Paste wird mit einer Vorrichtung gemäß Abbildung portionsweise in die Kavität eines Zahnes gebracht und bei einer Anpreßkraft von 5 N (mindestens 3 N) und einer Ultraschallfrequenz von 28 Kilohertz verfestigt. Nach 10 Minuten ist die Kavität gefüllt. Nach dem Polieren erhält man eine Oberflächenhärte von 40 Vickers.

0151758

84 106 DT

Degussa Aktiengesellschaft,
Weissfrauenstraße 9, 6000 Frankfurt/Main

Patentansprüche:

1. Verfahren zur Herstellung von Füllungen in Zähnen
   durch Einbringung von Goldpulver in die Kavität
   und anschließender mechanischer Verfestigung,
   dadurch gekennzeichnet,
   daß ein plättchenförmiges Goldpulver verwendet
   wird, das mit einem bei 20 bis 45° C sich verflüssigenden, plastischen organischen Bindemittel
   zu einer Paste verarbeitet wird, und daß die mechanische Verfestigung unter Einwirkung von Ultraschall erfolgt.

2. Verfahren zur Herstellung von Füllungen nach Anspruch 1,
   dadurch gekennzeichnet,
   daß als Bindemittel Polyäthylenglycol mit einem
   Molekulargewicht von 600 bis 1500 in Mengen von
   0,5 bis 5 Gew% verwendet wird.

3. Verfahren zur Herstellung von Füllungen nach Anspruch 1 und 2,
   dadurch gekennzeichnet,
   daß die Goldplättchen eine Größe von 5 bis 100 $\mu$m
   und eine Dicke von 0,1 bis 5 $\mu$m aufweisen.

4. Vorrichtung zur Herstellung von Füllungen in Zähnen nach Anspruch 1 bis 3, bestehend aus einem Ultraschallerzeuger mit stabförmiger Sonotrode, dadurch gekennzeichnet, daß die Sonotrode (2) von einer vor- und zurückschiebbaren Hülse (3) umgeben ist.

Fig.1